# EUROPEAN PATENT APPLICATION

(11) **EP 4 450 482 A1**
(43) Date of publication of application: **23.10.2024**
(21) Application number: 23305585.4
(22) Date of filing: 18.04.2023
(51) Int. Cl.: C07C 17/08, C07C 29/62, C07C 41/22, C07C 45/63, C07C 51/363, C07C 67/287

(54) **HYDROCHLORINATION OF ALKENES UNDER MILD CONDITIONS**

(71) Applicant: Centre National de la Recherche Scientifique, 75016 Paris (FR); Université de Rennes, 35042 Rennes (FR); Ecole Nationale Supérieure de Chimie de Rennes, 35700 Rennes (FR)
(72) Inventor: MÜLLER, Daniel, 35220 Saint-Jean-sur-Vilaine (FR); OLIVIER, M. Arnaud, 76000 Rouen (FR)
(74) Representative: Novagraaf Technologies

(57) **Abstract**

Process for the hydrochlorination of alkenes, comprising the following steps:
a)- preparing an organic solution A of a compound (I) comprising at least one alkene function by dissolving it potentially in an organic solvent;
b)- mixing the solution A with a aqueous solution B comprising hydrochloric acid;
c)- stirring the mixture comprising solution A and solution B at stirring speed of at least 500 rpm, and at a temperature in the range from -5 to 105°C for at least half an hour.

## Description

### Technical field

The present invention belongs to the field of the preparation of alkyl chlorides, and relates more specifically to a process to prepare such alkyl chlorides.

In the description below, references between [] refer to the list of references at the end of the examples.

### Technical background

Alkyl chlorides are undoubtedly one of the most important classes of organic compounds. They are commonly encountered in pharmaceuticals, agrochemicals and natural products. However, and more importantly, their potential to serve as electrophiles and after metal insertion as nucleophiles renders their chemistry extremely rich. The synthesis of alkyl chlorides mainly relies on functional group transformations of alcohols. Hydrochlorination of feedstock olefins is a highly attractive alternative but was somewhat hampered in the past by low reactions rates, a limited functional group tolerance and the lack of control concerning the product distribution.

The hydrochlorination of alkenes has a long history and is often utilized as an example in many organic textbooks to explain the Markovnikow rule. However, in practice only certain alkenes (e.g. trisubstituted ones) readily undergo hydrochlorination when exposed to HCl gas. Others, such as monosubstituted or 1,2-disubstituted alkenes react very sluggishly.

The hydrochlorination of alkenes can be separated into two major categories, the nucleophilic and radical addition pathway. The radical hydrochlorination has appeared relatively recently with a first report of Gaspar et al. in **[1].** Concerning the nucleophilic hydrochlorination, it is initiated by the protonation of the alkene followed by the trapping on the cationic intermediate by a chloride anion. When using HCl gas one needs to break the H-Cl bond (427 kJ/mol) which is stronger than a C-C bond (347 kJ/mol). This explains why additives such as FeCl₃, AlCl₃ or water which can favor the dissociation of H-Cl have an accelerating effect on the reaction, as described in ref. **[2].** The same is true for polar solvents which can stabilize the cationic intermediates.

Concerning the hydrochlorination of alkenes using HCl gas by bubbling it into or onto solutions of the corresponding neat alkenes, even though the hydrochlorination of alkene like propene is an exothermic process, the reaction with HCl gas does not take place at pressures of 1 atm or less **[2]**; and requires to work at a higher pressure and the reaction is sometimes difficult to implement in particular on an industrial scale.

Interestingly, hydrochloric acid, a widely available bulk chemical has been rarely employed for hydrochlorination reactions. The two major studies by Landini et al. **[3]** and Tanemura et al. **[4]** using phase transfer catalyst or silica gel activation demonstrated that hydrochlorination reactions can be carried out with hydrochloric acid, though necessitating in many cases several days for complete conversion.

To avoid the inconveniences of the background art, investigations have to be made to obtain secondary and tertiary alkyl chlorides through a simple hydrochlorination procedure avoiding the use of HCl gas, under atmospheric pressure and relatively short reaction times.

### Detailed description of the invention

The applicant has developed a new synthetic method or process for the preparation of alkyl chloride compounds that solves all the problems listed above.

The present invention thus deals with a process for the hydrochlorination of alkenes involving a hydrochloric acid solution and taking place in preferably less than 24 hours, ideally less than 12 hours.

The invention therefore relates to a process for the hydrochlorination of alkenes, comprising the following steps:
a)- preparing an organic solution A of a compound (I) comprising at least one alkene function by dissolving it potentially in an organic solvent;
b)- mixing the solution A with a aqueous solution B comprising hydrochloric acid;
c)- stirring the mixture comprising solution A and solution B at a stirring speed of at least 500 rpm, and at a temperature in the range from -5 to 105°C for at least half an hour, and preferably less than one day.

In step a) the organic solvent is used preferably if the compound is a solid. Nevertheless, it is also possible to avoid the solvent even if the alkene is a solid, providing in this case slower reaction.

Preferably, the compound (I) is a solid compound.

Advantageously, the ratio of hydrochloric acid over each alkene function of compound (I), in the mixture comprising solution A and solution B, corresponds to at least 1.2 equivalents of hydrochloric acid for one alkene function of the compound A, preferably at least 6 equivalents of hydrochloric acid for one alkene function of the compound A, and more preferably at least 12 equivalents of hydrochloric acid for one alkene function of compound A.

Advantageously, the compound (I) contains one alkene function and the solution B is a hydrochloric solution in the range of 33 - 37%. Hydrochloric acid is a solution of HCl gas in water. Commercially avaible hydrochloric acid has a maximum concentration of 37% (fuming hydrochloric acid, 12 M), and is easier to handle in comparison to HCl gas.

Advantageously, the stirring speed is of at least 1000 rpm, preferably 1150 rpm.

Advantageously, the stirring speed is from 1250 to 1750 rpm, preferably 1350 to 1650, and most preferably from 1450 to 1550 rpm. Rotation rates of 1500 rpm (revolutions per minute) are not accessible with all kind of stirring plates, and it is used preferably the stirring plate IKA^{®} RCT basic IKAMAG^{™} safety control.

Vigorous stirring typically from 500 to 2000 rpm is preferably required on a 1-1000 mmol scale of compound (I) for efficient reactions. Larger scale reactions, typically when the quantity of compound (I) is higher than 1 mol, can achieve similar conversions with lower stirring rates, i.e. 100 and 500 rpm. This is due to the increased surface of the aqueous and organic phase in a large reactor.

Advantageously, the temperature of the reaction in step c)- is from 10 to 90°C; preferably from 20 to 80°C; and most preferably from 30 to 70°C.

Advantageously, the reaction time at step c)- is in the range of 55 min to 8 h, preferably in the range of 2 to 7 h, and most preferably in the range of 3 to 6 h.

Advantageously, at step b)- an other acid is added to the mixture, preferably selected among a bronsted acid and a lewis acid, the bronsted acid being advantageously an organic acid and the lewis acid being a metal chloride. The organic acid is preferably a carboxylic acid is selected preferably in between AcOH (acetic acid), monochloroacetic acid, dichloroacetic acid, trichloroacetic acid and TFA (trifluoroacetic acid), and the metal chloride is chosen from FeCl₃, InCl₃, SbCl₅ and ZnCl₂, most preferably FeCl₃.

Advantageously, the compound (I) has a molecular structure according to the formula represented below: 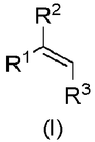 wherein R1, R2, R3 groups independently represents H, a cyclic or acyclic, substituted or unsubstituted, linear or branched C₁₋₁₂alkyl, C₂₋₁₂alkenyl, C₂₋₁₂alkynyl, C₁₋₁₂heteroalkyl, C₂₋₁₂heteroalkenyl, C₂₋₁₂heteroalkynyl, substituted or unsubstituted C₆₋₁₀aryl or C₄₋₁₀heteroaryl group; -OR^{A1}; - C(=O)R^{A1}; or -SR^{A1}; wherein each occurrence of R^{A1} independently represents hydrogen or a cyclic or acyclic, substituted or unsubstituted, linear or branched C₁₋₁₂alkyl, C₂₋₁₂alkenyl, C₂₋₁₂alkynyl, C₁₋₁₂heteroalkyl, C₂₋₁₂heteroalkenyl, C₂₋₁₂heteroalkynyl, C₆₋₁₀aryl or C₄₋₁₀heteroaryl group; wherein each of the foregoing alkyl, alkenyl, alkynyl, heteroalkyl, heteroalkenyl, heteroalkynyl, aryl or heteroaryl group may optionally bear one or more substituents selected from F; Cl; Br; I; -NO₂; -CN; -CF₃; - CH₂CF₃; CHCl₂; or -GR^{B1} wherein G is -O-, -S-, -NR^{C1}-, -C(=O)-, -S(=O)-, -SO₂-, -C(=O)O-, -C(=O)NR^{C1}-, -OC(=O)-, -NR^{C1}C(=O)-, wherein each occurrence of R^{B1} and R^{C1} independently represents independently represents hydrogen, or a C₁₋₁₂alkyl, C₂₋₁₂alkenyl, C₂₋₁₂alkynyl, C₁₋₁₂haloalkyl, C₂₋₁₂haloalkenyl, C₂₋₁₂haloalkynyl, C₁₋₁₂heteroalkyl, C₂₋₁₂heteroalkenyl, C₂₋₁₂heteroalkynyl, C₆₋₁₀aryl or C₄₋₁₀heteroaryl group.

Advantageously, the R1 group and R2 group are connected together to form a cycle, and compound (I) is preferably selected among

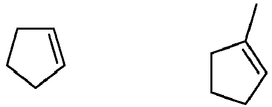

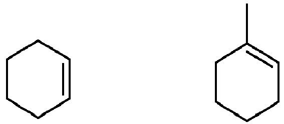

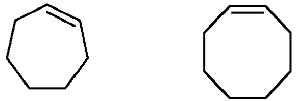

Advantageously, the compound (I) is selected among:

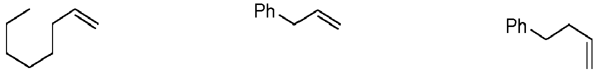

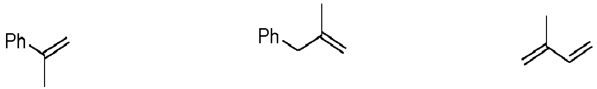

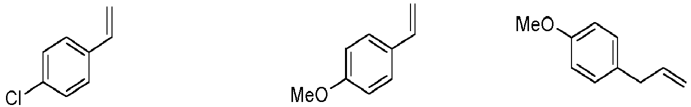

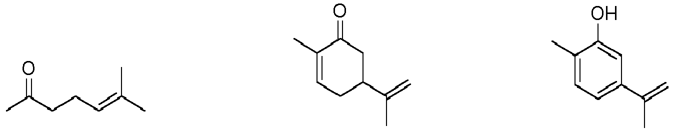

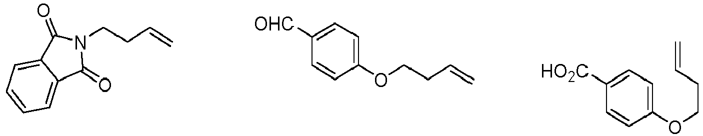

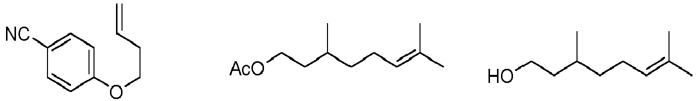

### Experimental part

### Devices and protocols

NMR spectra were recorded on a Bruker AV III 300 spectrometer operating at 300 MHz for 1H, equipped with a BBFO. All experiments were carried out at 25 °C, and references for chemical shifts are external. Coupling constants J were calculated in hertz (Hz). Multiplicity are annotated as s (singlet), d (doublet), t (triplet), m (multiplet), or o (overlapping signals). The conversion in equivalents of HCl is according to the following: 1 mL of HCl = 12 mmol of HCl; for example of a dosage 1mL/mmol corresponds to 12 equivalents of HCl with respect to the committed alkene function. Entry 1 in table 1, thus corresponds to 1.2 equivalents of HCl.

### Example 1: study of the role of stoichiometry

**Table 1: Optimization of reaction conditions for the hydrochlorination of alkene 1^{a}**

| **A)** | 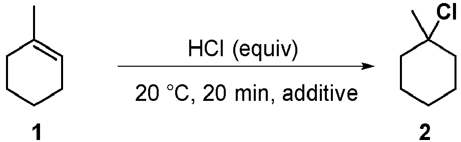 | | | |
|---|---|---|---|---|
| entry | HCl (mL/mmol) | additive (mL/mmol) | rpm^{b} | yield **(2)** (%)^{c} |
| 1 | 0.1 | none | 1500 | 11 |
| 2 | 0.5 | none | 1500 | 61 |
| 3 | 1.0 | none | 1500 | 81 |
| 4 | 2.0 | none | 1500 | 84 |
| 5 | 0.5 | AcOH (0.5 mL) | 1500 | 96 (81)^{d} |
| 6 | 1.0 | AcOH (0.25 mL) | 1500 | 87 |
| 7 | 1.0 | AcOH (0.5 mL) | 1500 | 90 |
| 8 | 1.0 | AcOH (1.0 mL) | 1500 | 98 |
| 9 | 2.0 | AcOH (1.0 mL) | 1500 | 97 |
| 10 | 1.0 | AcOH (1.0 mL) | 0 | 5 |
| 11 | 1.0 | AcOH (1.0 mL) | 500 | 8 |
| 12 | 1.0 | AcOH (1.0 mL) | 1000 | 16 |

Annex concerning Table 1: (a)Reactions performed a 1.0 mmol scale with 37% hydrochloric acid. (b)Revolutions per minute. (c)Yield determined by GC with decane as internal standard; no side-products were observed by GC. (d)Isolated yield on a 100 mmol scale, 2 hours reaction time; volatile product.

The first experiment afforded the product within 20 minutes albeit with relatively low yield (entry 1). Increasing the amount of hydrochloric acid from 0.1 mL to 2.0 mL / mmol of alkene let to a GC-yield of 84% (entry 4). The inventors have shown that the addition of 0.5 mL of acetic acid increased the yield from 61 to 96% (entries 2 and 5). They have also demonstrated that further variation of the amounts of hydrochloric acid and acetic acid showed that equal volumes of both reagents work best (entries 5 and 8).

Carrying out a large-scale reaction (100 mmol of **1**) afford an isolated pure alkyl chloride **2** in 81% yield. In this case conditions shown in entry 5 have been performed with half of the volumes of HCl and AcOH compared to entry 8. From the beginning on they worked under highly agitated conditions (1500 rpm), when changing the rotation rate to 0, 500 and 1000 rpm a drastic drop in GC-yield is observed (entries 10-12).

### Detail of the experimental conditions, provided for entry 6 table 1:

A one dram vial (4 mL) was charged with a stirring bar, 1-Methyl-1-cyclohexene (118 µL, 98 mg, 1.0 mmol, 1.0 equiv), n-decane (97.5 µL, 71.2 mg, 0.5 mmol, 0.5 equiv.), AcOH (0.25 mL), and HCl (37%, 1.0 mL). Immediately, after addition of HCl (37%) the vial was placed on a IKA RCT basic stirring plate which was already stirring at a rate of 1500 rpm. After 20 minutes of stirring at 20 °C the vial was removed from the stirring plate and 4 µL of the oily phase floating on top of the acidic phase was subjected to GC-analysis. GC-traces were recorded on a Agilent 8890 instrument equipped with a HP-5 column (30 m × 320 µm × 0.25 µm) as stationary phase. The following temperature protocol was used: 70 °C initial temperature (Hold time 3 min), Ramp 1 (10 °C/ min) from 70 -130 °C, then Ramp 2 (20 °C/ min ) from 130 °C - 300 °C (Hold time 3 minutes). Flow rate (H2) = 3.5 mL / min; Average velocity = 63.72 cm / sec. Integration of the signal of the internal standard (decane) and the corresponding product 1-chloro-1-methylcyclohexane (**2**) indicated a GC-yield of **2** of 87%.

### Example 2: study of different alkenes involved in the hydrochlorination process

The hydrochlorination of several other alkenes (Scheme 1) is also investigated. Hydrochlorination of trisubstituted alkenes proceeded smoothly, affording tertiary chlorides **4** and **5** in good yields. It is noteworthy that tertiary benzylic chloride **5** is thermally unstable and gave elimination product during attempted distillation. As expected cyclooctene reacted more sluggishly and required prolonged reaction times and heating to achieve full conversion. Interestingly, this reaction allows to access high-value added products such as chlorocyclooctane **6,** in only one step from cyclooctene, an inexpensive bulk chemical. The robustness of the hydrochlorination protocol was demonstrated for the synthesis of **7** from styrene on a 500 mmol scale with an excellent 97% yield (see scheme 1).

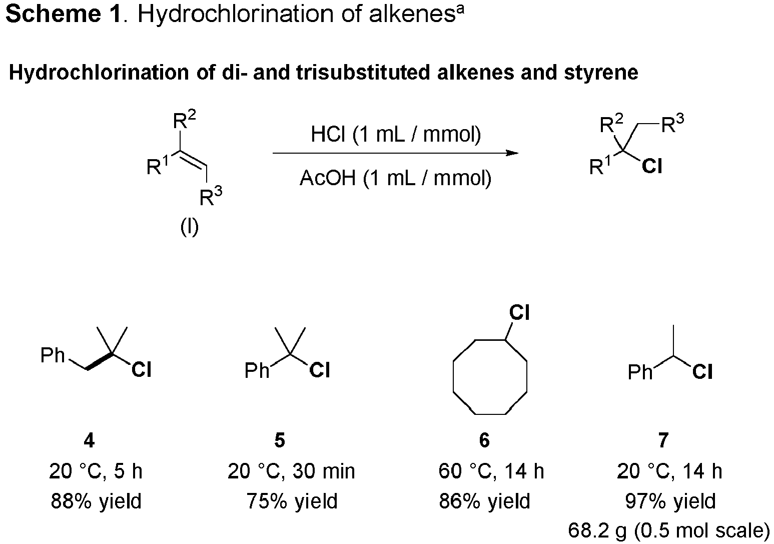

Annex - scheme 1 :(a)For all reactions 37% hydrochloric acid was used and the reactions were stirred at 1500 rpm.

### Example 3: study of the rate acceleration by acidic additives

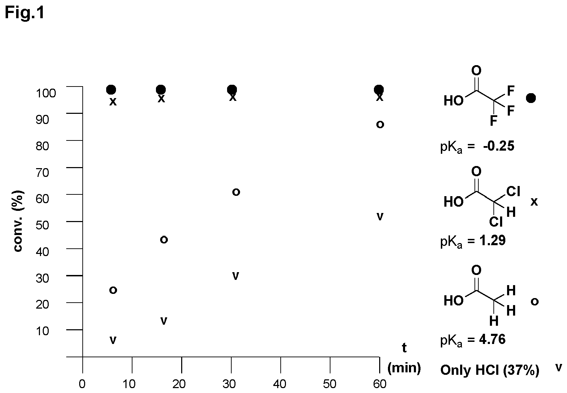

The hydrochlorination of styrene also served as a benchmark reaction in order to evaluate the influence of several carboxylic acids on the reaction rate (Scheme 2 and Figure 1). A strong dependence of the reaction rate on the pKₐ of the corresponding carboxylic acid is observed. Trifluoracetic acid and dichloroacetic acid gave >90% conversion within less than 5 minutes at room temperature which is an outstanding result in comparison to previous results reported in the literature (in general several hours reaction time are required).

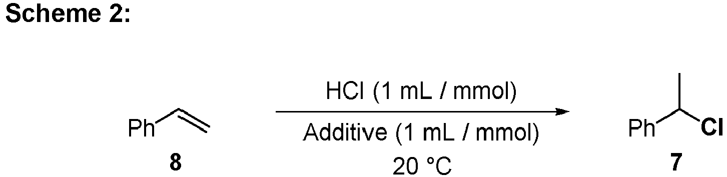

### Example 4: study of other alkenes involved in the hydrochlorination process

Hydrochlorination of 1-octene **10** and allylbenzene **14** gave significant amounts of acetate **13** and **17** which are very slowly transformed into the corresponding chlorides **11** and **15** (see Table 2). Indeed, the displacement of acetate **13** into 2-chlorooctane **11** is a very slow process requiring 14 hours at 80 °C for 64% conversion.

**Table 2: Hydrochlorination of 1-octene 10 and allyl benzene 14^{a}**

| **A) Hydrochlorination of 1-octene** | | | | |
|---|---|---|---|---|
| 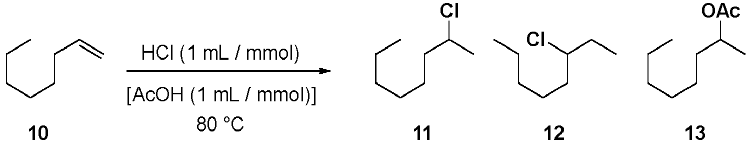 | | | | |
| **Conditions** | **10** (%)^{b} | **[11+12]** (%)^{b} | **11:12**^{c} | **[11+12]** : **13**^{c} |
| No acetic acid, 0.5 h | 92 | 6 | 79:21 | -- |
| No acetic acid, 14 h | 31 | 67 | 81:19 | -- |
| With acetic acid, 0.5 h | 78 | 17 | 95:5 | 75:25 |
| With acetic acid, 6 h | 2 | 83 | 87:13 | 85:15 |
| With acetic acid, 14 h | 0 | 99 | 81:19 | 99:1 |

| **B) Hydrochlorination of allyl benzene** | | | | |
|---|---|---|---|---|
| 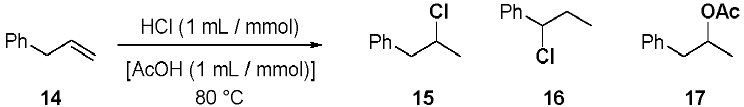 | | | | |
| **Conditions** | **14** (%)**^{b}** | **[15+16]** (%)**^{b}** | **15:16^{c}** | **[15+16]** : **17**^{c} |
| No acetic acid, 4 h | 17 | 79 | 88:12 | -- |
| No acetic acid, 14 h | 1 | 96 | 88:12 | -- |
| With acetic acid, 4 h | 1 | 88 | 89:11 | 89:11 |
| With acetic acid, 14 h | 0 | 90 | 90:10 | 95:5 |

Annex - table 2: (a)All reactions performed a 1.0 mmol scale with 1500 rpm stirring rate. (b)GC area percentage. (c)Determined by 1H-NMR analysis of the crude reaction mixture.

The inventors demonstrate that unexpectedly, the acceleration of the reaction of monosubstituted non-activated alkenes is clearly not the result of acetate formation and subsequent displacement. They hence postulate that the role of acetic acid is that of a proton transfer reagent. Acetic acid is miscible with hydrochloric acid and thus protonates partially by HCl and then probably delivers the proton unto the alkene in the organic phase.

### Example 5: study of the role of other additives

Trifluoro acetic acid and FeCl₃ promoted the reaction of allyl benzene at room temperature with almost complete conversion within as few as 30 minutes in the case of FeCl₃ (Table 3, entry 6). With both additives (TFA and FeCl₃) a multitude of unidentified trace side products were observed by GC-analysis, all with significantly higher retention times. The regioselectivity increased with reaction time and that with both, CF₃CO₂H and FeCl₃ as additive (Table 3).

**Table 3: Evaluation of several additives for the hydrochlorination of allylbenzene^{a}**

| 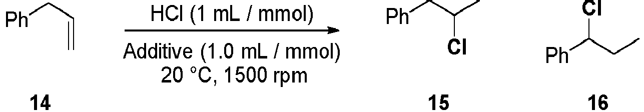 | | | | | | |
|---|---|---|---|---|---|---|
| entry | Additive | t [h] | **14** [%]^{b} | **15+16** [%]^{b} | **15:16** [%]^{c} | **Other** [%]^{b} |
| 1 | AcOH | 14 | 84 | 15 | 92:8 | 1 |
| 2 | CF₃CO₂H | 0.5 | 81 | 18 | 85:15 | 1 |
| 3 | CF₃CO₂H | 6 | 4 | **87** | 90:10 | 9 |
| 4 | CF₃CO₂H | 14 | 1 | 85 | 94:6 | 15 |
| 5 | CF₃CO₂H | 48 | 0 | 84 | 97:3 | 16 |
| 6 | FeCl₃ (7.0 equiv) | 0.5 | 3 | 82 | 91:9 | 15 |
| 7 | FeCl₃ (7.0 equiv) | 6 | 0 | 80 | >98:2 | 20 |

Annex - table 3: (a)All reactions performed a 1.0 mmol scale with 1500 rpm stirring rate. (b)GC area percentage. (c)Determined by 1H-NMR analysis of the crude reaction mixture.

In the case of allyl benzene, it is demonstrated that prolonged reaction times increases the regioselectivity.

### Example 6: study of functional group tolerance

- para-chloro substituted styrene afforded the desired product **20** in 90% yield. It is noteworthy that several reported protocols in the literature failed for this particular styrene or styrenes in general **[1, 5].**
- para-methoxy styrene, a strongly activated alkene, was submitted to the hydrochlorination protocol according to the invention. The standard conditions (1 mL/mmol HCl and AcOH) afforded full conversion in less than 15 minutes at room temperature, but only a gel like product was obtained. Dilution of the styrene in dichloromethane and only 5 equivalents of hydrochloric afforded the product in 42% NMR-yield accompanied with what we believe to be a oligo- or polymer side-product.
- In accordance with the results obtained with allylbenzene, 4-allyl anisole gave pure **22** with FeCl₃ as additive and subsequent distillation in reasonable yield.
- Ketones tolerate the relatively harsh reaction conditions, and the corresponding chloride **23** was isolated in 57% yield.
- carvone give an inseparable **24:25** mixture of the desired product **24** and eugenol **25.**
- Phtalimid and aldehyde substituted alkenes give the corresponding products **26** and **27** respectively without formation of a significant amount of regiomers.
- The hydrochlorination of a benzonitrile substrate gave concomitant nitrile hydrolysis and decomposition resulting in a low yield of carboxylic acid **28.**
- Iron trichloride as additive preserved the nitrile group and we were able to isolate the desired product **29** in 62% yield.
- Citronellol give the hydrochlorinated citronellol **31** in high yield. This demonstrates that the process according to the invention does not require protection of primary alcohols when reactive alkenes are hydrochlorinated.
- Hydrochlorination of isoprene **32** was followed by ¹H-NMR. After 30 minutes full consumption of isoprene was observed affording a 72:28 mixture of prenyl chloride **33** and dichloride **34** when carrying out the reaction with only hydrochloric acid (0.5 mL/ mmol). In contrast, the reaction with 0.5 mL / mmol TFA as additive gave 100% conversion of isoprene in only 5 minutes and a 8:92 mixture of **33:34** thus demonstrating the rate acceleration with trifluoro acetic acid as additive. On a large scale 400 mmol of isoprene was fully hydrochlorinated with hydrochloric acid (37%) to **34** in a 90% yield after 2 days of reaction. Pure hydrochloric acid was preferential in this case compared to TFA and acetic acid due to side-reactions with the two additives (see scheme 3).

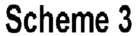

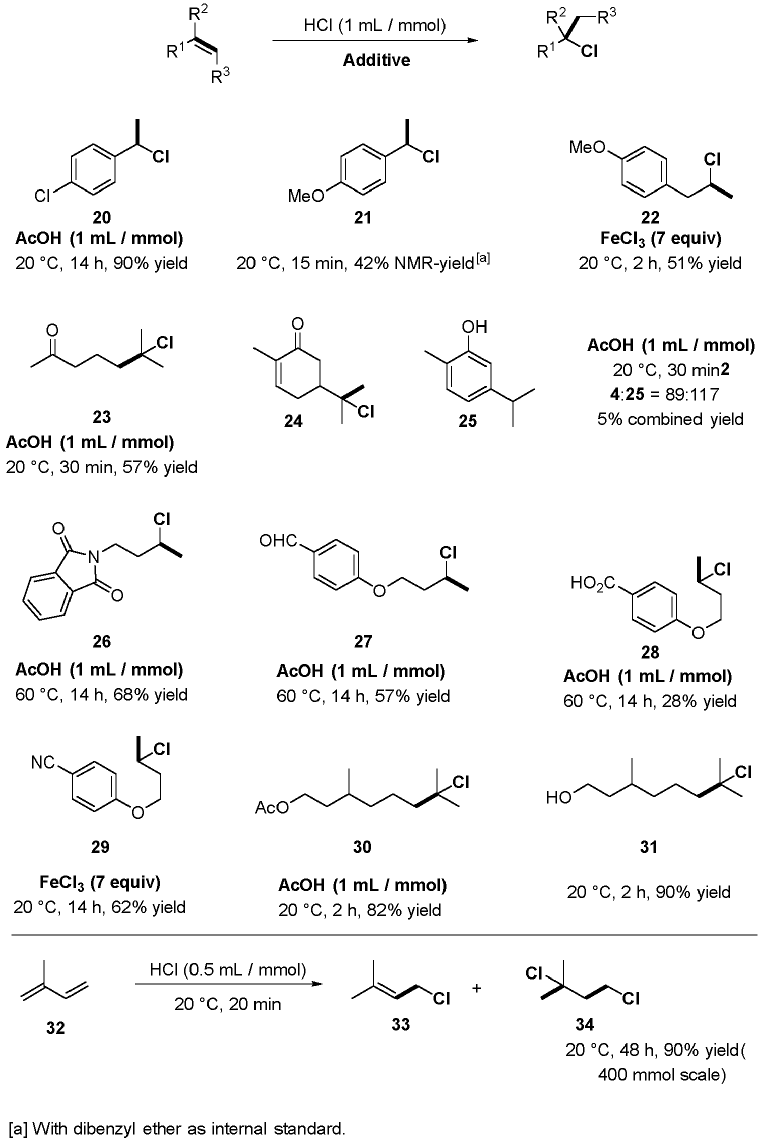

### Example 7: study of cyclic alkenes as reagent in the hydrochlorination process

The process according to the invention is also tested starting from other alkenes as shown in scheme 4. 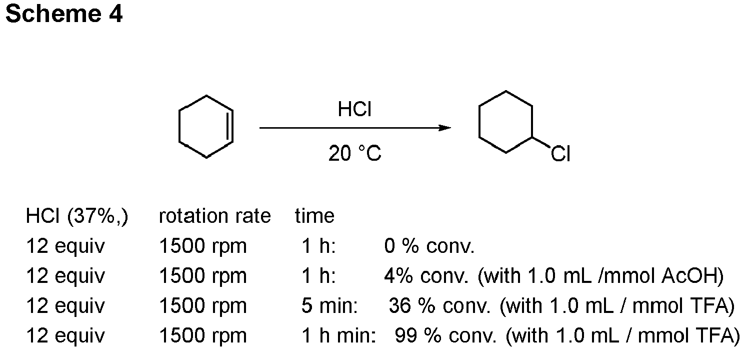 Clean reaction, TFA leads again to a dramatic rate increase (4% conv with AcOH, under identical conditions 99% conv. with TFA)

### List of references

[1] Gaspar, B.; Carreira, E. M. Catalytic Hydrochlorination of Unactivated Olefins with Para-Toluenesulfonyl Chloride. Angewandte Chemie International Edition 2008, 47 (31), 5758-5760. https://doi.org/10.1002/anie.200801760.
[2] US 6 617 479**.**
[3] Landini, D.; Rolla, F. Addition of Hydrohalogenic Acids to Alkenes in Aqueous-Organic, Two-Phase Systems in the Presence of Catalytic Amounts of Onium Salts. J. Org. Chem. 1980, 45 (17), 3527-3529_https://doi.org/10.1021/jo01305a038 ;
[4] Tanemura, K. Silica Gel-Mediated Hydrohalogenation of Unactivated Alkenes Using Hydrohalogenic Acids under Organic Solvent-Free Conditions. Tetrahedron Letters 2018, 59 (49), 4293-4298_https://doi.org/10.1016/j.tetlet.2018.10.043 ;
[5] Yadav, V. K.; Babu, K. G. A Remarkably Efficient Markovnikov Hydrochlorination of Olefins and Transformation of Nitriles into Imidates by Use of AcCI and an Alcohol. European Journal of Organic Chemistry 2005, 2005 (2), 452-456. https://doi.org/10.1002/ejoc.200400591;

## Claims

1. Process for the hydrochlorination of alkenes, comprising the following steps:
a)- preparing a organic solution A of a compound (I) comprising at least one alkene function by dissolving it potentially in an organic solvent;
b)- mixing the solution A with a aqueous solution B comprising hydrochloric acid;
c)- stirring the mixture comprising solution A and solution B at a stirring speed of at least 500 rpm, and at a temperature in the range from -5 to 105°C for at least half an hour, and preferably less than one day.

2. Process according to claim 1, wherein the ratio of hydrochloric acid over each alkene function of compound (I), in the mixture comprising solution A and solution B, corresponds to at least 1.2 equivalents of hydrochloric acid for one alkene function of the compound A, preferably at least 6 equivalents of hydrochloric acid for one alkene function of the compound A, and more preferably at least 12 equivalents of hydrochloric acid for one alkene function of compound A.

3. Process according to claim 1 or 2, wherein the compound (I) contains one alkene function and the solution B is a hydrochloric solution in the range of 33 - 37%.

4. Process according to anyone of claims 1 to 3, wherein the stirring speed is from 1250 to 1750 rpm, preferably 1350 to 1650, and most preferably from 1450 to 1550 rpm.

5. Process according to anyone of claims 1 to 4, wherein the temperature of the reaction in step c)- is from 10 to 90°C; preferably from 20 to 80°C; and most preferably from 30 to 70°C.

6. Process according to anyone of claims 1 to 5, wherein the reaction time at step c)- is in the range of 55 min to 8 h, preferably in the range of 2 to 7 h, and most preferably in the range of 3 to 6 h.

7. Process according to anyone of claims 1 to 6, wherein at step b)- an other acid is added to the mixture, preferably selected among an organic acid and a metal chloride.

8. Process according to anyone of claims 1 to 7, wherein the compound (I) has a molecular structure according to the formula represented below: 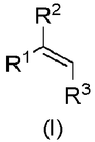 wherein R1, R2, R3 groups independently represents H, a cyclic or acyclic, substituted or unsubstituted, linear or branched C₁₋₁₂alkyl, C₂₋₁₂alkenyl, C₂₋₁₂alkynyl, C₁₋₁₂heteroalkyl, C₂₋₁₂heteroalkenyl, C₂₋₁₂heteroalkynyl, substituted or unsubstituted C₆₋₁₀aryl or C₄₋₁₀heteroaryl group; -OR^{A1}; - C(=O)R^{A1}; or -SR^{A1}; wherein each occurrence of R^{A1} independently represents hydrogen or a cyclic or acyclic, substituted or unsubstituted, linear or branched C₁₋₁₂alkyl, C₂₋₁₂alkenyl, C₂₋₁₂alkynyl, C₁₋₁₂heteroalkyl, C₂₋₁₂heteroalkenyl, C₂₋₁₂heteroalkynyl, C₆₋₁₀aryl or C₄₋₁₀heteroaryl group; wherein each of the foregoing alkyl, alkenyl, alkynyl, heteroalkyl, heteroalkenyl, heteroalkynyl, aryl or heteroaryl group may optionally bear one or more substituents selected from F; Cl; Br; I; -NO₂; -CN; -CF₃; - CH₂CF₃; CHCl₂; or -GR^{B1} wherein G is -O-, -S-, -NR^{C1}-, -C(=O)-, -S(=O)-, -SO₂-, -C(=O)O-, -C(=O)NR^{C1}-, -OC(=O)-, -NR^{C1}C(=O)-, wherein each occurrence of R^{B1} and R^{C1} independently represents independently represents hydrogen, or a C₁₋₁₂alkyl, C₂₋₁₂alkenyl, C₂₋₁₂alkynyl, C₁₋₁₂haloalkyl, C₂₋₁₂haloalkenyl, C₂₋₁₂haloalkynyl, C₁₋₁₂heteroalkyl, C₂₋₁₂heteroalkenyl, C₂₋₁₂heteroalkynyl, C₆₋₁₀aryl or C₄₋₁₀heteroaryl group.

9. Process according to claim 8, wherein the R1 group and R2 group are connected together to form a cycle, and compound (I) is preferably selected among:

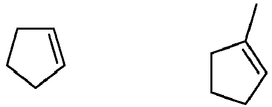

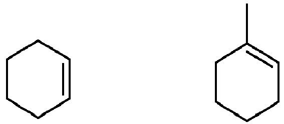

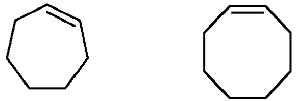

10. Process according to claim 8, wherein the compound (I) is selected among:
